# EUROPEAN PATENT SPECIFICATION

(11) **EP 0 999 872 B1**
(45) Date of publication and mention of the grant of the patent: **07.12.2005**
(21) Application number: 99920422.5
(22) Date of filing: 10.05.1999
(51) Int. Cl.: A61N 1/36

(54) **PERCUTANEOUS INTRAMUSCULAR STIMULATION SYSTEM**
PERKUTANES INTRAMUSKULÄRES STIMULATIONSSYSTEM
SYSTEME DE STIMULATION INTRAMUSCULAIRE PERCUTANE

(30) Priority: 03.06.1998 US 89994
(43) Date of publication of application: 17.05.2000
(73) Proprietor: Neurocontrol Corporation, Valley View, OH 44125 (US)
(72) Inventor: FANG, Zi-Ping, Mayfield Village, OH 44143 (US); POURMEHDI, Soheyl, Beachwood, OH 44122 (US)
(74) Representative: Fuchs Mehler Weiss & Fritzsche
(86) International application number: PCT/US1999/010221
(87) International publication number: WO 1999/062594

(56) References cited:
- EP-A- 0 165 049
- GB-A- 2 123 698
- GB-A- 2 223 949
- US-A- 4 408 609
- US-A- 5 092 329
- US-A- 5 167 229

## Description

The present invention relates to the art of therapeutic neuromuscular stimulation. It finds particular application for use by human patients who are paralyzed or partially paralyzed due to cerebrovascular accidents such as stroke or the like. The invention is useful for retarding or preventing muscle disuse atrophy, maintaining extremity range-of-motion, facilitating voluntary motor function, relaxing spastic muscles, increasing blood flow to select muscles, and the like.

An estimated 555,000 persons are disabled each year in the United States of America by cerebrovascular accidents (CVA) such as stroke. Many of these patients are left with partial or complete paralysis of an extremity. For example, subluxation (incomplete dislocation) of the shoulder joint is a common occurrence and has been associated with chronic and debilitating pain among stroke survivors. In stroke survivors experiencing shoulder pain, motor recovery is frequently poor and rehabilitation is impaired. Thus, the patient may not achieve his/her maximum functional potential and independence. Therefore, prevention and treatment of subluxation in stroke patients is a priority.

There is a general acknowledgment by healthcare professionals of the need for improvement in the prevention and treatment of shoulder subluxation. Conventional intervention includes the use of orthotic devices, such as slings and supports, to immobilize the joint in an attempt to maintain normal anatomic alignment. The effectiveness of these orthotic devices varies with the individual. Also, many authorities consider the use of slings and arm supports to be controversial or even contraindicated because of the potential complications from immobilization including disuse atrophy and further disabling contractures.

Surface, i.e., transcutaneous, electrical muscular stimulation has been used therapeutically for the treatment of shoulder subluxation and associated pain, as well as for other therapeutic uses. Therapeutic transcutaneous stimulation has not been widely accepted in general because of stimulation-induced pain and discomfort, poor muscle selectivity, and difficulty in daily management of electrodes. In addition to these electrode-related problems, commercially available stimulators are relatively bulky, have high energy consumption, and use cumbersome connecting wires.

In light of the foregoing deficiencies, transcutaneous stimulation systems are typically limited to two stimulation output channels. The electrodes mounted on the surface of the patient's skin are not able to select muscles to be stimulated with sufficient particularity and are not suitable for stimulation of the deeper muscle tissue of the patient as required for effective therapy. Any attempt to use greater than two surface electrodes on a particular region of a patient's body is likely to result in suboptimal stimulation due to poor muscle selection. Further, transcutaneous muscle stimulation via surface electrodes commonly induces pain and discomfort.

Studies suggest that conventional interventions are not effective in preventing or reducing long term pain or disability. Therefore, it has been deemed desirable to develop a percutaneous, i.e., through the skin, neuromuscular stimulation system that utilizes temporarily implanted, intramuscular stimulation electrodes connected by percutaneous electrode leads to an external and portable pulse generator.

The present invention departs from a percutaneous, intramuscular stimulation system for therapeutic electrical stimulation of select muscles of a patient as it is known from US-A-5167229.

Further similar muscle stimulator systems for producing an electrical output pulse train for providing functional electrical stimulation of muscles are known from EP-A-0165049 and US-A-4408609.

Departing from this prior art it is the object of the present invention to devise a percutaneous, intramuscular stimulation system which allows in an easy way a quick and proper adjustment of the stimulation pulse train parameters.

This object is achieved by the characterizing features of claim 1. Further advantageous embodiments of the inventive stimulation system may be taken from the dependent claims.

Still further benefits and advantages of the present invention will become apparent to those of ordinary skill in the art upon reading and understanding the following detailed description of the preferred embodiments.

### Brief Description of the Drawings

The invention may take form in various components and arrangements of components, and in various steps and arrangements of steps. The drawings are only for purposes of illustrating preferred embodiments, and are not to be construed as limiting the invention.
FIGURE 1A is a front elevational view of a portable, programmable stimulation pulse train generator in accordance with the present invention;
FIGURES 1B - 1D are top, bottom, and right-side elevational views of the stimulation pulse train generator of FIGURE 1A;
FIGURE 2 illustrates a preferred intramuscular electrode and percutaneous electrode lead;
FIGURE 3 diagrammatically illustrates the structure and operation of the percutaneous intramuscular stimulation system in accordance with the present invention;
FIGURE 3A diagrammatically illustrates a preferred pulse amplitude/duration controller, current driver, and impedance detector circuit in accordance with the present invention; and,
FIGURE 4 graphically illustrates the stimulation paradigm of the percutaneous intramuscular stimulation system in accordance with the present invention.

### Detailed Description of the Preferred Embodiments

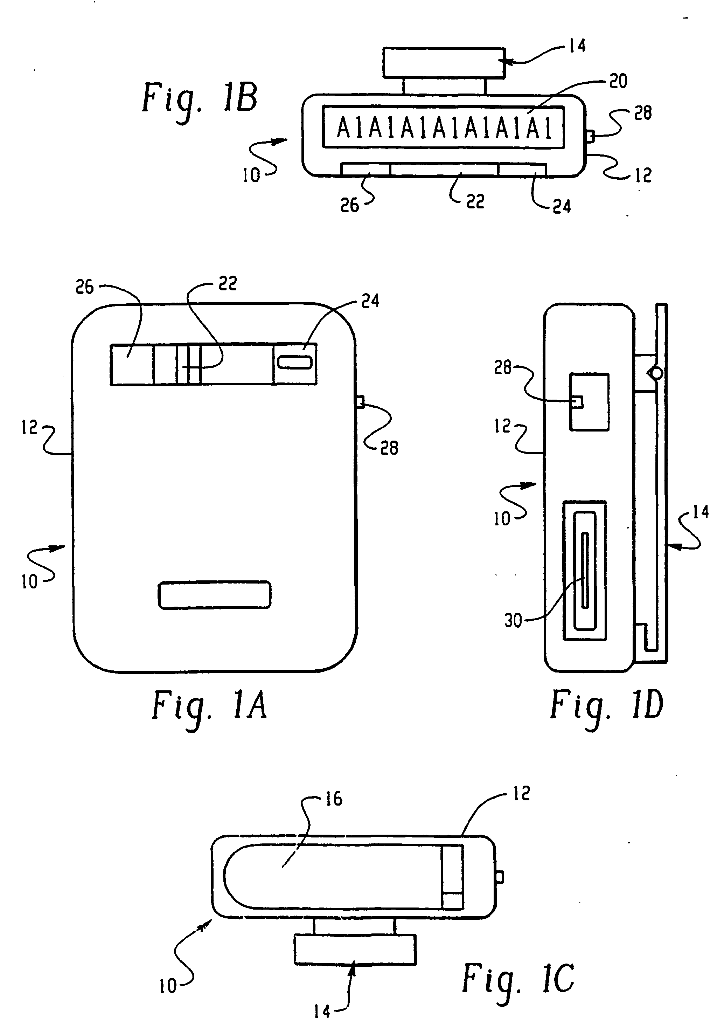

With reference to FIGURES 1A-1D, the percutaneous, intramuscular stimulation system in accordance with the present invention includes an electrical stimulation pulse generator 10. The pulse generator 10 includes a lightweight, durable plastic housing 12 fabricated from a suitable plastic or the like. The case 12 includes a clip 14 that allows the pulse generator 10 to be releasably connected to a patient's belt, other clothing, or any other convenient location. The case 12 also includes a releasable battery access cover 16.

For output of visual data to a patient or clinician operating the stimulation system, a visual display 20 is provided. The display 20 is preferably provided by a liquid crystal display, but any other suitable display means may alternatively be used. An audio output device, such as a beeper 22 is also provided.

For user control, adjustment, and selection of operational parameters, the stimulation pulse generator 10 includes means for input of data. Preferably, the pulse generator 10 includes an increment switch 24, a decrement switch 26, and a select or "enter" switch 28. The increment and decrement switches 24, 26 are used to cycle through operational modes or patterns and stimulation parameters displayed on the display 20, while the select switch 28 is used to select a particular displayed operational pattern or stimulation parameter. The select switch 28 also acts as a power on/off toggle switch. By choosing the appropriate mode, the select switch 28 can be selectively armed as a "hot button." During adjustment of stimulation pulse train parameters, a clinician is able to activate the hot button to test, instantaneously, the effect of the selected stimulation pulse train parameters on the patient's muscles. This facilitates the quick and proper adjustment of the stimulation pulse train parameters without requiring the clinician to exit the setup procedure menu of the stimulation pulse generator 10.

For output of electrical stimulation pulse train signals, the pulse train generator 10 includes an external connection socket 30 that mates with a connector of an electrode cable assembly (not shown) to interconnect the pulse generator 10 with a plurality of intramuscular electrodes via percutaneous electrode leads. More particularly, the cable assembly connected to the socket 30 includes a second connector on a distal end that mates with a connector attached to the proximal end of each of the percutaneous stimulation electrode leads and a reference electrode lead.

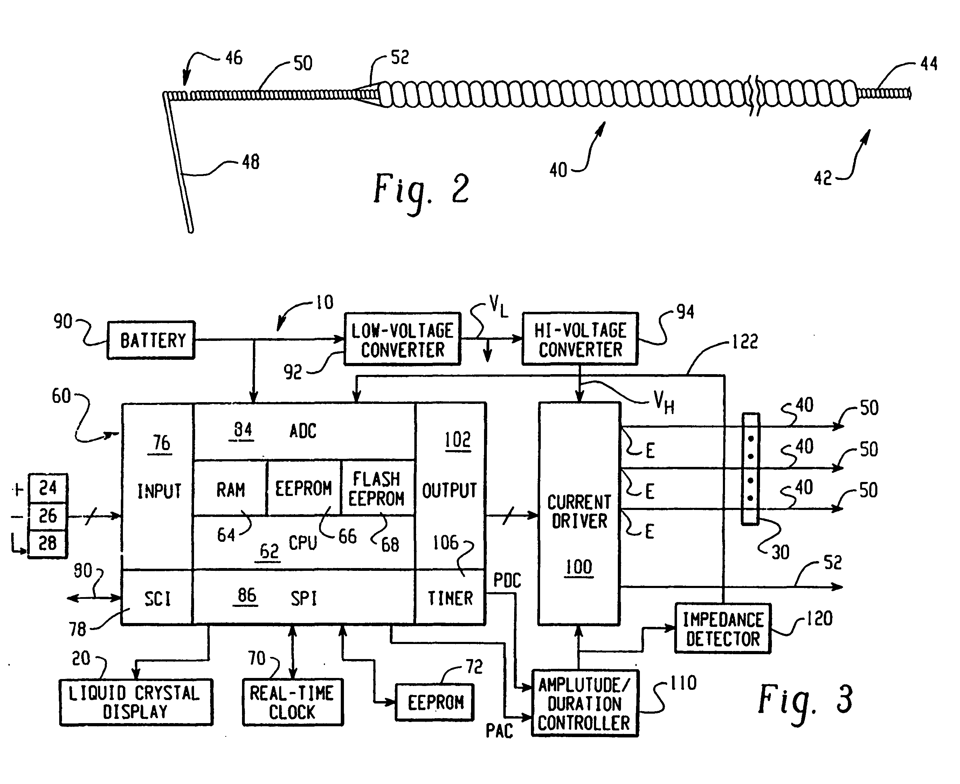

A preferred intramuscular electrode and percutaneous lead are shown in FIGURE 2. The electrode lead 40 is fabricated from a 7-strand stainless steel wire insulated with a biocompatible polymer. Each individual wire strand has a diameter of 34 µm and the insulated multi-strand lead wire has a diameter of 250 µm. The insulated wire is formed into a spiral or helix as has been found preferred to accommodate high dynamic stress upon muscle flexion and extension, while simultaneously retaining low susceptibility to fatigue. The outer diameter of the helically formed electrode lead 40 is approximately 580 µm and it may be encased or filled with silicone or the like.

As mentioned above, a proximal end 44 of each of the plurality of intramuscular electrode lead wires 40 is located exterior to the patient's body when in use. The proximal end 44 includes a deinsulated length for connection to an electrical connector in combination with the remainder of the electrode leads. The distal end 46 of each lead 40, which is inserted directly into muscle tissue, also includes a deinsulated length which acts as the stimulation electrode 50. It is preferred that at least a portion of the deinsulated length be bent or otherwise deformed into a barb 48 to anchor the electrode in the selected muscle tissue. A taper 52, made from silicone adhesive or the like, is formed between the deinsulated distal end 50 and the insulated portion of the lead 40 to reduce stress concentration.

Unlike surface electrodes which are applied to the surface of the patient's skin using an adhesive, each of the plurality of percutaneous electrodes 50 is surgically implanted into select patient muscle tissue, and the associated electrode lead 40 exits the patient percutaneously, i.e., through the skin, for connection to the stimulation pulse generator 10. Preferably, each of the electrodes 50 is implanted into the select muscles by use of a hypodermic needle. Once all of the electrodes are implanted as desired, their proximal ends are crimped into a common connector that mates with the cable assembly which is, in turn, connected to the pulse generator 10 through the connection socket 30.

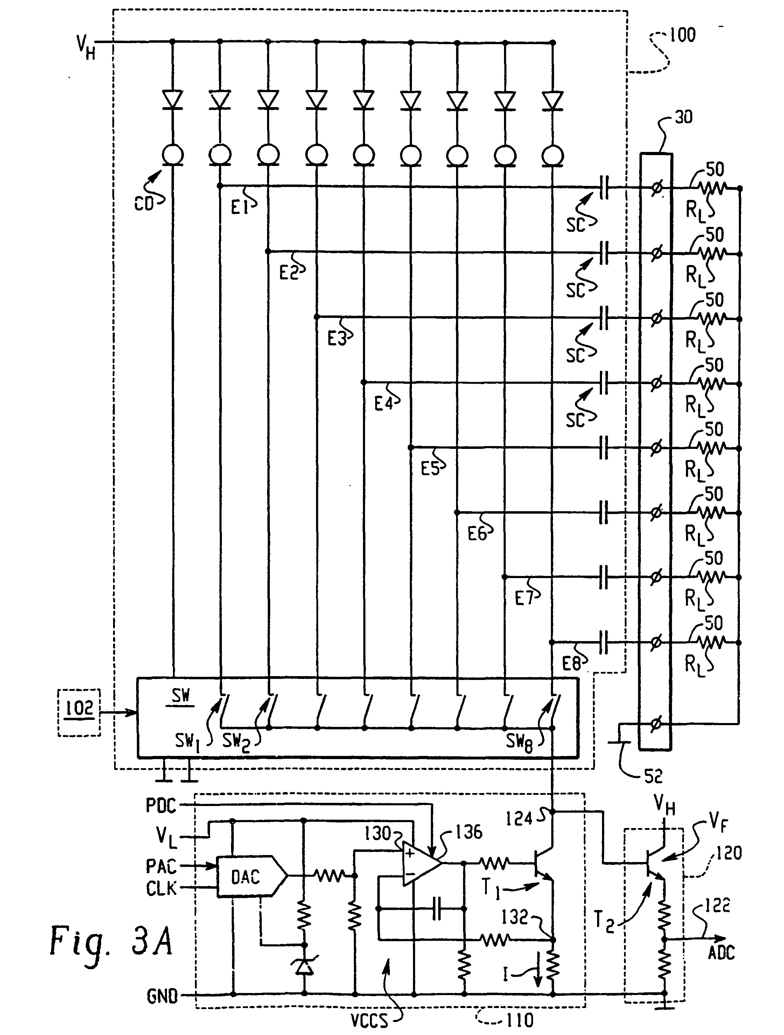

FIGURE 3 diagrammatically illustrates the overall percutaneous, intramuscular stimulation system in accordance with the present invention. Unlike surface stimulation systems which exhibit poor muscle selectivity and are, thus, typically limited to two stimulation electrodes and channels, the present percutaneous, intramuscular stimulation system allows for precise muscle selection and use of three or more stimulation electrodes and channels. The preferred system in accordance with the present invention uses up to eight or more intramuscular electrodes 50, each connected to an independent electrode stimulation channel E, and a single reference electrode 52 which may be either an intramuscular or surface electrode. Those of ordinary skill in the art will also recognize that the use of intramuscular electrodes allows for selection and stimulation of deep muscle tissue not practicable by surface stimulation.

The stimulation pulse generator 10 comprises a microprocessor-based stimulation pulse generator circuit 60. The preferred microcontroller is a Motorola 68HC12, although other suitable microcontrollers may be used without departing from the scope and intent of the invention. The circuit 60 comprises a central processing unit (CPU) 62 for performing all necessary operations. Random access memory (RAM) 64 is present for temporary storage of operational data as needed by the CPU 62. A first nonvolatile memory means, such as electrically erasable programmable read only memory (EEPROM) 66, provides nonvolatile storage as needed for operational instructions or other information, although the first nonvolatile memory means may not necessarily be used. Preferably, flash EPROM 68 (rather than write-once EPROM) is provided for storage of software operating instructions. Use of flash EPROM 68 facilitates periodic, unlimited upgrade of the software operating instructions.

In order to log or record patient usage of the stimulation pulse generator 10, the stimulation circuit 60 includes a real-time clock 70 along with a second nonvolatile memory means such as EEPROM 72 to provide sufficient nonvolatile storage for recording and timestamping a patient's use of the system. A clinician is thereafter able to access the EEPROM 72 to review the patient's use of the system to ensure patient compliance with the prescribed therapeutic stimulation protocol. Preferably, the second nonvolatile memory 72 also provides storage for all patient-specific stimulation protocols.

The increment, decrement, and select user input switches 24,26,28 are operatively connected into the circuit 60 via an input stage 76. In addition, a serial communication interface (SCI) 78 provides means for selectively connecting an external device, such as a computer, as needed by way of an RS-232 connection 80 or the like for data upload and download. An analog-to-digital converter 84 performs all analog-to-digital conversion of data as needed for processing in the circuit 60. A serial peripheral interface (SPI) 86 provides means for connecting peripheral components, such as the display 20, the clock 70, the EEPROM 72, and other components to the microcontroller.

Electrical potential or energy is supplied to the circuit 60 by a battery 90, preferably AA in size and ranging from 1.0 - 1.6 volts. A low-voltage dc-dc converter 92 adjusts the voltage supplied by the battery 90 to a select level V_{L}, preferably 3.3 volts. To minimize depletion of the battery during periods of inactivity of the pulse generator 10, the circuit 60 is programmed to automatically power-down after a select duration of inactivity. Those skilled in the art will recognize that the RAM 64 provides volatile storage, and the storage means 66,68,72 provide nonvolatile storage to prevent loss of data upon interruption of power to the circuit 60 through malfunction, battery depletion, or the like.

The output V_{L} of the low-voltage dc-dc converter 92 is also supplied to a high-voltage dc-dc converter 94 which steps-up the voltage to at least 30 volts. The high-voltage output V_{H} of the dc-dc converter 94 provides the electrical potential for the stimulation pulse train signals transmitted to the plurality of intramuscular electrodes 50 through a current driver 100. More particularly, an output means 102 of the circuit 60 provides channel selection input to the current driver 100 to control the transmission of the high-voltage electrical potential from the driver 100 to the selected electrodes 50 on a selected one of the plurality of stimulation output channels E. Although only three output channels E are illustrated, those skilled in the art will recognize that a greater number of output channels may be provided. Preferably, eight output channels E are provided.

The electrical current passes between the selected electrodes 50 and the reference electrode 52. A pulse duration timer 106 provides timing input PDC as determined by the CPU 62 to the pulse amplitude/duration controller 110 to control the duration of each stimulation pulse. Likewise, the CPU 62 provides a pulse amplitude control signal PAC to the circuit 110 by way of the serial peripheral interface 86 to control the amplitude of each stimulation pulse. One suitable circuit means for output of stimulation pulses as described above is in accordance with that described in U.S. Patent 5,167,229.

In order to ensure that an electrode lead is properly transmitting the stimulation pulse train signals to the select muscle tissue, an impedance detection circuit 120 monitors the impedance of each electrode lead 40. The impedance detection circuit 120 provides an analog impedance feedback signal 122 to the analog-to-digital converter 84 where it is converted into digital data for input to the CPU 62. Upon breakage of a lead 40 or other malfunction, the impedance detection circuit detects a change in impedance, and correspondingly changes the impedance feedback signal 122. The impedance feedback signal 122 allows the microcontroller to interrupt stimulation and/or generate and error signal to a patient or clinician.

FIGURE 3A is a somewhat simplified diagrammatic illustration of a most preferred current driver circuit 100, pulse amplitude/duration control circuit 110, and impedance detection circuit 120. The illustrated current driver circuit 100 implements eight output channels E1-E8, each of which is connected to an electrode 50 implanted in muscle tissue for passing electrical current through the muscle tissue in conjunction with the reference electrode 52. Accordingly, the patient muscle tissue and implanted electrodes 50 are illustrated as a load R_{L} connected to each channel E1-E8.

Each output channel E1-E8 includes independent electrical charge storage means such as a capacitor SC which is charged to the high voltage V_{H} through a respective current limiting diode CD. To generate a stimulation pulse, the microcontroller output circuit 102 provides channel select input data to switch means SW, such as an integrated circuit analog switch component, as to the particular channel E1-E8 on which the pulse is to be passed. Switch means SW closes the selected switch SW₁-SW₈ accordingly. The microcontroller also provides a pulse amplitude control signal PAC into a voltage-controlled current source VCCS. The pulse amplitude control signal PAC is converted into an analog signal at 130 by the digital-to-analog converter DAC. The analog signal at 130 is supplied to an operational amplifier 136 which, in conjunction with the transistor T₁, provides a constant current output I from the voltage-controlled current source VCCS. Of course, those of ordinary skill in the art will recognize that the particular magnitude of the constant current I is varied depending upon the magnitude of the voltage signal at 130 input to the OP-AMP 136, i.e., the circuit VCCS is provided such that the voltage at point 132 seeks the magnitude of the voltage at point 130. As such, the pulse amplitude control signal PAC controls the magnitude of the current I, and the circuit VCCS ensures that the current I is constant at that select level as dictated by the pulse amplitude control input PAC. For stimulation of human muscle, it is preferable that the current I be within an approximate range of 1mA -20mA.

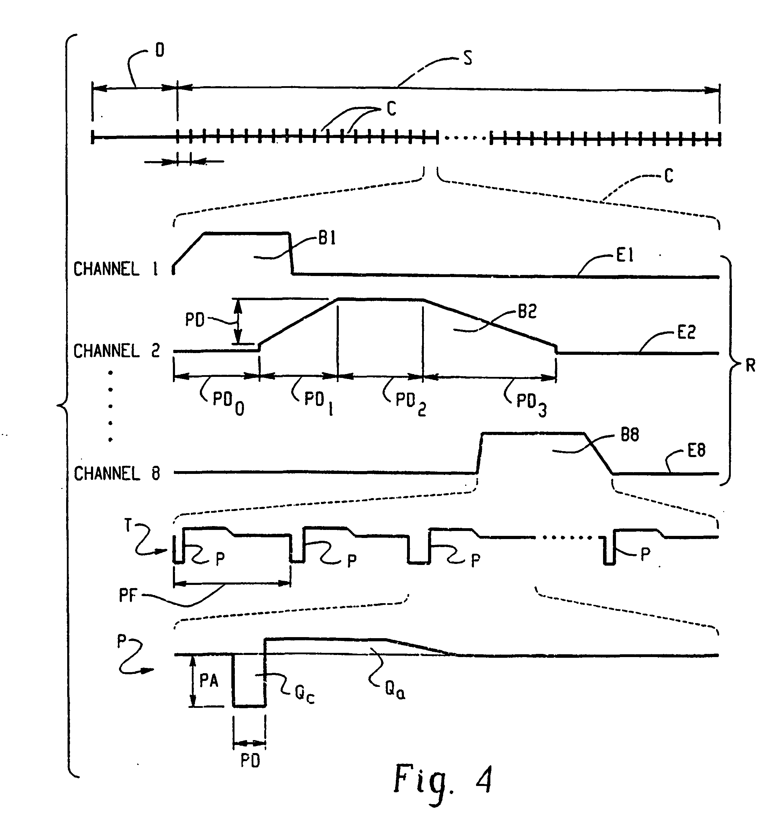

Upon closing one of switches SW₁-SW₈, the relevant capacitor SC discharges and induces the current I as controlled by the pulse amplitude control signal PAC and a pulse duration control signal PDC. The constant current I passes between the reference electrode 52 and the relevant one of the electrodes 50 to provide a cathodic pulse phase Q_{c} (see FIGURE 4). The pulse duration PD of the phase Q_{c} is controlled by the microcontroller through a pulse duration control signal PDC output by the timer circuit 106 into the pulse amplitude/duration control circuit 110. In particular, the pulse duration control signal PDC is input to a shut-down input of the OP-AMP 136 to selectively enable or blank the output of the OP-AMP 136 as desired, and, thus, allow or stop the flow of current I between the electrodes 50,52.

Upon completion of the cathodic phase Q_{c} as controlled by the pulse duration control signal PDC, the discharged capacitor SC recharges upon opening of the formerly closed one of the switches SW₁-Sw₈. The flow of recharging current to the capacitor SC results in a reverse current flow between the relevant electrode 50 and the reference electrode 52, thus defining an anodic pulse phase Qₐ. The current amplitude in the anodic pulse phase Qₐ is limited, preferably to 0.5mA, by the current limiting diodes CD. Of course, the duration of the anodic phase is determined by the charging time of the capacitor SC, and current flow is blocked upon the capacitor becoming fully charged. It should be recognized that the interval between successive pulses or pulse frequency PF is controlled by the CPU 62 directly through output of the channel select, pulse amplitude, and pulse duration control signals as described at a desired frequency PF.

The impedance detection circuit 120 "detects" the voltage on the active channel E1-E8 (i.e., the channel on which a pulse is being passed) through implementation of a high-impedance voltage follower circuit VF using a transistor T₂. Accordingly, it will be recognized that the voltage at points 122 and 124 will move together. Accordingly, for example, in the event of breakage of an electrode lead 40, a drop in voltage at point 124 will cause a corresponding drop in voltage at point 122. The voltage signal at point 122 is fed back to the microcontroller analog-to-digital converter 84 for interpretation by the CPU 62 in accordance with stored expected values indicating preferred impedance ranges. At the same time, the CPU 62 knows which switch SW₁-SW₈ is closed. Therefore, the CPU 62 is able to determine the channel E1-E8 on which the lead breakage occurred.

The preferred stimulus pulse train paradigm is graphically illustrated in FIGURE 4. A preferred design implements up to 4 independent preprogrammed patterns. For each pattern, a stimulation session S is pre-programmed into the stimulator circuit 60 by a clinician through use of the input means 24,26,28. Each session S has a maximum session duration of approximately 9 hours, and a session starting delay D. The maximum session starting delay D is approximately 1 hour. The session starting delay D allows a patient to select automatic stimulation session start at some future time. Within each session S, a plurality of stimulation cycles C are programmed for stimulation of selected muscles. Preferably, each stimulation cycle ranges from 2 - 100 seconds in duration.

With continuing reference to FIGURE 4, a stimulus pulse train T includes a plurality of successive stimulus pulses P. As is described above and in the aforementioned U.S. Patent 5,167,229, each stimulus pulse P is current-regulated and biphasic, i.e., comprises a cathodic charge phase Q_{c} and an anodic charge phase Qₐ. The magnitude of the cathodic charge phase Q_{c} is equal to the magnitude of the anodic charge phase Qₐ. The current-regulated, biphasic pulses P provide for consistent muscle recruitment along with minimal tissue damage and electrode corrosion.

Each pulse P is defined by an adjustable pulse amplitude PA and an adjustable pulse duration PD. The pulse frequency PF is also adjustable. Further, the pulse amplitude PA, pulse duration PD, and pulse frequency PF are independently adjustable for each stimulation channel E. The amplitude of the anodic charge phase Qₐ is preferably fixed at 0.5 mA, but may be adjusted if desired.

Pulse "ramping" is used at the beginning and end of each stimulation pulse train T to generate smooth muscle contraction. Ramping is defined herein as the gradual change in cathodic pulse charge magnitude by varying at least one of the pulse amplitude PA and pulse duration PD. In FIGURE 4, the preferred ramping configuration is illustrated in greater detail. As mentioned, each of the plurality of stimulation leads/electrodes 40,50 is connected to the pulse generator circuit 60 via a stimulation pulse channel E. As illustrated in FIGURE 4, eight stimulation pulse channels E1,E2,E8 are provided to independently drive up to eight intramuscular electrodes 50. Stimulation pulse trains transmitted on each channel E1-E8 are transmitted within or in accordance with a stimulation pulse train envelope B1-B8, respectively. The characteristics of each envelope B1-B8 are independently adjustable by a clinician for each channel E1-E8. Referring particularly to the envelope B2 for the channel E2, each envelope B1-B8 is defined by a delay or "off" phase PD₀ where no pulses are delivered to the electrode connected to the subject channel, i.e., the pulses have a pulse duration PD of 0. Thereafter, according to the parameters programmed into the circuit 60 by a clinician, the pulse duration PD of each pulse P is increased or "ramped-up" over time during a "ramp-up" phase PD₁ from a minimum initial value (e.g., 5 µsec) to a programmed maximum value. In a pulse duration "hold" phase PD₂, the pulse duration PD remains constant at the maximum programmed value. Finally, during a pulse duration"ramp-down" phase PD₃, the pulse duration PD of each pulse P is decreased over time to lessen the charge delivered to the electrode 50.

This "ramping up" and "ramping down" is illustrated even further with reference to the stimulation pulse train T which is provided in correspondence with the envelope B8 of the channel E8. In accordance with the envelope B8, the pulses P of the pulse train T first gradually increase in pulse duration PD, then maintain the maximum pulse duration PD for a select duration, and finally gradually decrease in pulse duration PD.

As mentioned, the pulse amplitude PA, pulse duration PD, pulse frequency PF, and envelope B1-B8 are user-adjustable for every stimulation channel E, independently of the other channels. Preferably, the stimulation pulse generator circuit 60 is pre-programmed with up to four stimulation patterns which will allow a patient to select the prescribed one of the patterns as required during therapy.

Most preferably, the pulse generator 10 includes at least up to eight stimulation pulse channels E. The stimulation pulse trains T of each channel E are sequentially or substantially simultaneously transmitted to their respective electrodes 50. The pulse frequency PF is preferably adjustable within the range of approximately 5Hz to approximately 50Hz; the cathodic amplitude PA is preferably adjustable within the range of approximately 1mA to approximately 20mA; and, the pulse duration PD is preferably adjustable in the range of approximately 5 µsec to approximately 200µsec, for a maximum of approximately 250 pulses per second delivered by the circuit 60.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A percutaneous, intramuscular stimulation system for therapeutic electrical stimulation of select muscles of a patient, said stimulation system comprising:
a plurality of intramuscular stimulation electrodes (50) for implantation directly into selected muscles of a patient, each electrode (50) including an insulated percutaneous lead (40);
an external battery-operated, microprocessor-based stimulation pulse train generator (10) for generating select electrical stimulation pulse trains, said external pulse train generation including:
a plurality of electrical stimulation pulse train output channels connected respectively to said plurality of percutaneous electrode leads (40);
an input device (24, 26, 28) for operator selection of stimulation pulse train parameters for each of said stimulation pulse train output channels independently of the other channels, said stimulation pulse train parameters including at least a pulse amplitude and pulse duration for stimulation pulses of said stimulation pulse train, and an interpulse interval between successive pulses of said stimulation pulse train defining a pulse frequency;
a visual output display (20) which provides visual output data to an operator of the pulse train generator (10), said visual output data including at least said stimulation pulse train parameters for each of said stimulation pulse train output channels;
a non-volatile memory (66, 68) which stores said stimulation pulse train parameters for each of said plurality of stimulation pulse train output channels; and,
a pulse train generation system for generating stimulation pulse train signals with the select pulse train parameters on each of said plurality of stimulation pulse train output channels, so that stimulus pulses of said pulse train signals having the select stimulation pulse train parameters pass between the intramuscular electrodes (50) respectively connected to said stimulation pulse train output channels and a reference electrode (52), **characterized in that** said input device (24, 26, 28) includes an increment switch (24) and a decrement switch (26) used to cycle through patterns and stimulation parameters displayed on said display (20) and further includes a select switch (28) used to select a particular displayed pattern or stimulation parameter, so that a desired stimulation pulse train envelope for each of said plurality of stimulation pulse train output channels can be selected, wherein each stimulation pulse train envelope is controlling a stimulation pulse train signal ramping paradigm including at least an initial ramp-up phase of a first select duration, an intermediate hold phase of a second select duration, and a terminal ramp-down phase of a third select duration, wherein, for each of said plurality of channels, stimulation pulses of said stimulation pulse train signal transmitted therein progressively increase in charge during said ramp-up phase, maintain a substantially constant charge during said hold phase, and progressively decrease in charge during said ramp down phase.

2. The percutaneous, intramuscular stimulation system as set forth in claim 1 wherein said pulse train generator (10) further includes:
a data recorder (60) for recording data describing prior use of said pulse train generator (10) in said non-volatile memory (66, 68), said data recorder (60) connected to said visual output display (20) so that an operator of said pulse train generator (10) can selectively visually display said pulse train generator (10) use data using said visual output display (20) to ensure compliance with prescribed stimulation therapy.

3. The percutaneous, intramuscular stimulation system as set forth in claim 2 wherein said data recorder (60) further includes a real-time clock (70) to provide time data to be recorded with said pulse train generator (10) use data.

4. The percutaneous, intramuscular stimulation system as set forth in claim 1 wherein said charge of said stimulation pulses is varied by controlling at least one of the pulse duration and pulse amplitude of each of said pulses.

5. The percutaneous, intramuscular stimulation system as set forth in claim 1 wherein said stimulation pulses are constant-current pulses having a cathodic phase (Q_{C}) and an anodic phase (Q_{A}) of opposite polarity but substantially equal charge.

6. The percutaneous, intramuscular stimulation system as set forth in claim 1 wherein said external pulse generator (10) further includes:
a low-voltage direct-current-to-direct-current converter (92) for connection to a battery (90) for converting electrical potential from the battery (90) into a select operating voltage for said pulse train generator (10); and,
a high-voltage direct-current-to-direct-current converter (94) connected to said low-voltage converter (92) for converting said operating voltage output by said low-voltage converter (94) into a stimulation voltage of at least 30 volts, said high-voltage converter (92) having an output of said stimulation voltage connected to said pulse train signal generation system.

7. The percutaneous, intramuscular stimulation system as set forth in claim 6 wherein said pulse train signal generation system includes:
a constant-current source (100) having an input connected to said stimulation voltage output of said high-voltage converter (94) and an output connected to each of said stimulation channels; and,
means for selectively connecting said constant-current source to each of said stimulation pulse train output channels in accordance with output channel select data received from output channel selection means to generate said stimulation pulse train signals on each of said output channels in accordance with said stored stimulus pulse train parameters for each of said plurality of channels.

8. The percutaneous, intramuscular stimulation system as set forth in claim 1 wherein said pulse train generator (10) further includes:
a switch (S_{W}) for instantaneously generating a stimulus pulse train signal on one of said plurality of output channels in accordance with selected stimulus pulse train parameters when said switch (S_{W}) is activated.

9. The percutaneous, intramuscular stimulation system as set forth in claim 1, wherein said pulse train generator (10) further includes:
a means (120) for measuring the impedance of each of said intramuscular electrodes (50) and associated percutaneous electrode leads (40), said impedance measuring means (120) providing feedback signal (122) to a central processing unit (62) of said pulse train generator (10) indicating impedance changes in said electrode (50) and associated electrode lead (40).

10. The percutaneous, intramuscular stimulation system as set forth in claim 1 wherein said non-volatile memory (66, 68) further includes stimulation session delay data indicating a select time interval after which a stimulation pulse train session is to begin in accordance with the stored stimulation pulse train parameters.

## Patentansprüche

1. Perkutanes, intramuskuläres Stimulationssystem zur therapeutischen elektrischen Stimulierung von ausgewählten Muskeln eines Patienten, wobei das Stimulationssystem umfasst:
mehrere intramuskuläre Stimulationselektroden (50) zur direkten Implantation in ausgewählten Muskeln eines Patienten, wobei jede Elektrode eine isolierte perkutane Leitung (40) umfasst;
einen externen batteriebetriebenen auf einem Mikroprozessor basierenden Stimulations-Impulszug-Generator (10) zur Erzeugung ausgewählter elektrischer Stimulations-Impuls-Züge, wobei der externe Impulszug-Generator umfasst:
mehrere elektrische Stimulations-Impulszug-Ausgangskanäle, die entsprechend an die mehreren perkutanen Elektrodenleitungen (40) angeschlossen sind;
eine Eingangseinrichtung (24, 26, 28) zur Operatorauswahl von Stimulations-Impulszug-Parametern für jeden der Stimulations-Impulszug-Ausgangskanäle unabhängig von den anderen Kanälen, wobei die Stimulations-Impulszug-Parameter wenigstens eine Impulsamplitude und eine Impulsdauer für die Stimulationsimpulse des Stimulations-Impulszuges und ein Zwischen-Impulsintervall zwischen aufeinander folgenden Impulsen des Stimulations-Impulszuges, die eine Impulsfrequenz vorgeben, umfassen;
ein visuelles Ausgabedisplay (20), welches visuelle Ausgangsdaten einem Operator des Impulszug-Generators vorgibt, wobei die visuellen Ausgangsdaten wenigstens die Stimulations-Impulszug-Parameter für jeden der Stimulations-Impulszug-Ausgangskanäle umfassen;
einen nicht-flüchtigen Speicher (66, 68), der Stimulations-Impulszug-Parameter für jeden der mehreren Stimulations-Impulszug-Ausgangskanäle speichert; und
ein Impulszug-Erzeugungssystem zur Erzeugung von Stimulations-Impulszug-Signalen mit den ausgewählten Impulszug-Parametern auf jedem der mehreren Stimulations-Impulszug-Ausgangskanäle, so dass Stimulationsimpulse der Impulszug-Signale mit den ausgewählten Stimulations-Impulszug-Parametem zwischen den intramuskulären Elektroden (50), die entsprechend an die Stimulations-Impulszug-Ausgangskanäle angeschlossen sind, und einer Referenzelektrode (50) verlaufen, **dadurch gekennzeichnet, dass** die Eingangseinrichtung (24, 26, 28) einen Erhöhungsschalter (24) und einen Erniedrigungsschalter (26) umfasst, die verwendet werden, um durch Muster und Stimulationsparameter zu schalten, die auf dem Display (20) angezeigt werden, und ferner einen Auswahlschalter (28) umfassen, der verwendet wird, um ein bestimmtes angezeigtes Muster oder einen Stimulationsparameter auszuwählen, so dass eine gewünschte Stimulations-Impulszug-Einhüllende für jeden der mehreren Stimulations-Impulszug-Ausgangskanäle ausgewählt werden kann, wobei jede Stimulations-Impulszug-Einhüllende ein Stimulations-Impulszug-Signal-Neigungsparadigma steuert, das wenigstens eine anfängliche Steigungsphase mit einer ersten ausgewählten Dauer, eine Zwischen-Haltephase mit einer zweiten ausgewählten Dauer und eine letztliche Abfallphase mit einer dritten ausgewählten Dauer umfasst, wobei für jeden der mehreren Kanäle, die in dem Stimulations-Impulszug-Signal übertragenen Stimulationsimpulse progressiv in der Ladung während der Steigungsphase anwachsen, eine im wesentlichen konstante Ladung während der Haltephase beibehalten und progressiv in der Ladung während der Abfallphase abnehmen.

2. Perkutanes, intramuskuläres Stimulationssystem, wie im Anspruch 1 wiedergegeben, wobei der Impulszug-Generator (10) ferner umfasst:
einen Datenrekorder (60) zur Aufzeichnung von Daten in dem nicht-flüchtigen Speicher (66, 68), die die vorherige Verwendung des Impulszug-Generators (60) beschreibt, wobei der Datenrekorder (60) an das visuelle Ausgabedisplay (20) angeschlossen ist, so dass ein Operator des Impulszug-Generators (10) selektiv visuell die von dem Impulszug-Generator (10) verwendeten Daten unter Verwendung des visuellen Ausgabedisplays (20) anzeigen kann, um die Übereinstimmung mit der vorgeschriebenen Stimulationstherapie sicherzustellen.

3. Perkutanes, intramuskuläres Stimulationssystem, wie im Anspruch 2 wiedergegeben, wobei der Datenrekorder (60) ferner einen Echtzeit-Takt (70) umfasst, um Zeittaktdaten vorzugeben, die mit den von dem Impulszug-Generator (10) verwendeten Daten aufgezeichnet werden.

4. Perkutanes, intramuskuläres Stimulationssystem, wie im Anspruch 1 wiedergegeben, wobei die Ladung der Stimulationsimpulse verändert wird durch Steuerung von wenigstens der Impulsdauer und der Impulsamplitude von jedem der Impulse.

5. Perkutanes, intramuskuläres Stimulationssystem, wie im Anspruch 1 wiedergegeben, wobei die Stimulationsimpulse von Konstantstrom-Impulse mit einer kathodischen Phase (Q_{C}) und einer anodischen Phase (Q_{A}) entgegengesetzter Polarität aber im wesentlichen gleicher Ladung sind.

6. Perkutanes, intramuskuläres Stimulationssystem, wie im Anspruch 1 wiedergegeben, wobei der externe Impulsgenerator (10) ferner umfasst:
einen Niedrigspannungs-Gleichstrom/Gleichstrom-Wandler (92) für den Anschluss an eine Batterie (90) zur Umwandlung eines elektrischen Potenziales von der Batterie (90) in eine ausgewählte Betriebsspannung für den Impulszug-Generator (10); und
einen Hochspannungs-Gleichstrom/Gleichstrom-Wandler (94), der an den Niedrigspannungs-Wandler (92) angeschlossen ist, um den Betriebsspannungsausgang von dem Niedrigspannungs-Wandler (92) in eine Stimulationsspannung von wenigstens 30 Volt umzuwandeln, wobei der Hochspannungs-Wandler (94) mit einem Ausgang der Stimulationsspannung an das Impulszug-Signalerzeugungssystem angeschlossen ist.

7. Perkutanes, intramuskuläres Stimulationssystem, wie im Anspruch 6 wiedergegeben, wobei das Impulszug-Signalerzeugungssystem umfasst:
eine Konstantstromquelle (100), die mit einem Eingang an den Stimulationsspannungsausgang des Hochspannungswandlers (94) und mit einem Ausgang an jeden der Stimulationskanäle angeschlossen ist; und
Mittel für die selektive Verbindung der Konstantstromquelle mit jedem der Stimulations-Impulszug-Ausgangskanäle in Übereinstimmung mit Ausgangskanal-Auswahldaten, die von einer Ausgangskanal-Auswahleinrichtung empfangen werden, um die Stimulations-Impulszug-Signale auf jedem der Ausgangskanäle gemäß den gespeicherten Stimulations-Impulszug-Parametem für jeden der mehreren Kanäle zu erzeugen.

8. Perkutanes, intramuskuläres Stimulationssystem, wie im Anspruch 1 wiedergegeben, wobei der Impulszug-Generator (10) ferner umfasst:
einen Schalter (S_{W}) für die augenblickliche Erzeugung eines Stimulations-Impulszug-Signals auf einem der mehreren Ausgangskanäle gemäß ausgewählter Stimulations-Impulszug-Parameter, wenn der Schalter (S_{W}) betätigt ist.

9. Perkutanes, intramuskuläres Stimulationssystem, wie im Anspruch 1 wiedergegeben, wobei der Impulszug-Generator (10) ferner umfasst:
eine Einrichtung (120) zur Messung der Impedanz einer jeder der intramuskulären Elektroden (50) und von zugeordneten perkutanen Elektrodenleitungen (40), wobei die Impedanz-Messeinrichtung (120) ein Rückmeldesignal (122) an eine zentrale Verarbeitungseinheit (62) des Impulszug-Generators (10) vorgibt, das Impedanzänderungen in der Elektrode (50) und der zugeordneten Elektrodenleitung (40) anzeigt.

10. Perkutanes, intramuskuläres Stimulationssystem, wie im Anspruch 1 wiedergegeben, wobei der nicht-flüchtige Speicher (66, 68) ferner Stimulationssitzungs-Verzögerungsdaten umfasst, die ein ausgewähltes Zeitintervall anzeigen, nach welchem eine Stimulations-Imputszug-Sitzung in Übereinstimmung mit den gespeicherten Stimulations-Impulszug-Parametern beginnen kann.

## Revendications

1. Système de stimulation intramusculaire percutané pour la stimulation électrique thérapeutique de muscles choisis d'un patient, ledit système de stimulation comprenant :
une pluralité d'électrodes (50) de stimulation intramusculaires destinées à être implantées directement dans les muscles choisis d'un patient, chaque électrode (50) comprenant un fil (40) percutané isolé ;
un générateur (10) de train d'impulsions de stimulation externe alimenté par batterie, à microprocesseur, destiné à générer des trains d'impulsions de stimulation électrique choisis, ledit générateur de trains d'impulsions externe comprenant :
une pluralité de canaux de sortie de train d'impulsions de stimulation électrique connectés respectivement à ladite pluralité de fils (40) pour électrodes percutanés ;
un dispositif d'entrée (24, 26, 28) pour que l'opérateur choisisse des paramètres de train d'impulsions de stimulation pour chacun des canaux de sortie de train d'impulsions de stimulation indépendamment des autres canaux, lesdits paramètres de train d'impulsions de stimulation comprenant au moins une amplitude d'impulsion et une durée d'impulsion pour les impulsions de stimulation dudit train d'impulsions de stimulation, et un intervalle entre impulsions entre les impulsions successives dudit train d'impulsions de stimulation définissant une fréquence d'impulsion ;
un affichage de sortie visuelle (20) qui fournit des données de sortie visuelle à un opérateur du générateur (10) de train d'impulsions, lesdites données de sortie visuelle comprenant au moins lesdits paramètres de train d'impulsions de stimulation pour chacun desdits canaux de sortie de train d'impulsions de stimulation ;
une mémoire non volatile (66, 68) qui enregistre lesdits paramètres de train d'impulsions de stimulation pour chacun desdits canaux de sortie de train d'impulsions de stimulation ; et
un système de génération de train d'impulsions destiné à générer des signaux de train d'impulsions de stimulation avec les paramètres de train d'impulsions choisis sur chaque canal de ladite pluralité de canaux de sortie de train d'impulsions de stimulation, de telle sorte que des impulsions de stimulus desdits signaux de train d'impulsions ayant les paramètres de train d'impulsions de stimulation choisis passent entre les électrodes intramusculaires (50) respectivement connectées auxdits canaux de sortie de train d'impulsions de stimulation et une électrode de référence (52), **caractérisé en ce que** ledit dispositif d'entrée (24, 26, 28) comprend un commutateur d'incrémentation (24) et un commutateur de décrémentation (26) utilisés pour effectuer des cycles à travers les motifs et les paramètres de stimulation affichés sur ledit affichage (20), et comprend en outre un sélecteur (28) utilisé pour sélectionner un motif ou un paramètre de stimulation affiché particulier, de telle sorte qu'une enveloppe de train d'impulsions de stimulation souhaitée pour chaque canal de ladite pluralité de canaux de sortie de train d'impulsions de stimulation puisse être choisie, dans lequel chaque enveloppe de train d'impulsions de stimulation contrôle un paradigme de rampage de signal de train d'impulsions de stimulation comprenant au moins une phase initiale d'accélération d'une première durée choisie, une phase intermédiaire d'attente d'une deuxième durée choisie, et une phase terminale de décélération d'une troisième durée choisie, dans lequel, pour chaque canal de ladite pluralité de canaux, les impulsions de stimulation dudit signal de train d'impulsions de stimulation transmis dans celui-ci augmentent progressivement du point de vue de la charge pendant ladite phase d'accélération, maintiennent une charge sensiblement constante pendant ladite phase d'attente, et diminuent progressivement du point de vue de la charge pendant ladite phase de décélération.

2. Système de stimulation intramusculaire percutané selon la revendication 1, dans lequel ledit générateur (10) de train d'impulsions comprend en outre :
un enregistreur de données (60) destiné à enregistrer les données qui décrivent l'utilisation antérieure dudit générateur (10) de train d'impulsions dans ladite mémoire non volatile (66, 68), ledit enregistreur de données (60) étant connecté audit affichage de sortie visuelle (20) de telle sorte qu'un opérateur dudit générateur (10) de train d'impulsions puisse afficher visuellement de manière sélective lesdites données d'utilisation du générateur (10) de train d'impulsions à l'aide dudit affichage de sortie visuelle (20) afin de garantir le respect de la thérapie de stimulation prescrite.

3. Système de stimulation intramusculaire percutané selon la revendication 2, dans lequel ledit enregistreur de données (60) comprend en outre une horloge temps réel (70) destinée à fournir des données temporelles destinées à être enregistrées avec lesdites données d'utilisation du générateur (10) de train d'impulsions.

4. Système de stimulation intramusculaire percutané selon la revendication 1, dans lequel ladite charge desdites impulsions de stimulation est modifiée en contrôlant au moins l'un des éléments parmi la durée d'impulsion et l'amplitude d'impulsion de chacune desdites impulsions.

5. Système de stimulation intramusculaire percutané selon la revendication 1, dans lequel lesdites impulsions de stimulation sont des impulsions à courant constant ayant une phase cathodique (Q_{C}) et une phase anodique (Q_{A}) de polarité opposée mais de charge sensiblement égale.

6. Système de stimulation intramusculaire percutané selon la revendication 1, dans lequel ledit générateur (10) d'impulsions externe comprend en outre :
un convertisseur (92) courant continu-courant continu basse tension destiné à être connecté à une batterie (90) afin de transformer le potentiel électrique provenant de la batterie (90) en une tension de fonctionnement choisie pour ledit générateur (10) de train d'impulsions ; et
un convertisseur (94) courant continu-courant continu haute tension connecté audit convertisseur (92) basse tension destiné à transformer ladite sortie de tension de fonctionnement par ledit convertisseur (94) basse tension en une tension de stimulation d'au moins 30 volts, ledit convertisseur (92) haute tension ayant une sortie de ladite tension de stimulation connectée audit système de génération de signal de train d'impulsions.

7. Système de stimulation intramusculaire percutané selon la revendication 6, dans lequel ledit système de génération de signal de train d'impulsions comprend :
une source (100) de courant constant ayant une entrée connectée à ladite sortie de tension de stimulation dudit convertisseur (94) haute tension et une sortie connectée à chacun desdits canaux de stimulation ; et
des moyens destinés à connecter de manière sélective ladite source de courant constant à chacun desdits canaux de sortie de train d'impulsions de stimulation en fonction des données de sélection de canal de sortie reçues à partir des moyens de sélection de canal de sortie afin de générer lesdits signaux de train d'impulsions de stimulation sur chacun desdits canaux de sortie en fonction desdits paramètres de train d'impulsions de stimulus enregistrés pour chaque canal de ladite pluralité de canaux.

8. Système de stimulation intramusculaire percutané selon la revendication 1, dans lequel ledit générateur (10) de train d'impulsions comprend en outre :
un commutateur (S_{W}) destiné à générer de manière instantanée un signal de train d'impulsions de stimulus sur l'un des canaux de ladite pluralité de canaux de sortie en fonction des paramètres de train d'impulsions de stimulus choisis lorsque ledit commutateur (S_{W}) est activé.

9. Système de stimulation intramusculaire percutané selon la revendication 1, dans lequel ledit générateur (10) de train d'impulsions comprend en outre :
un moyen (120) destiné à mesurer l'impédance de chacune desdites électrodes intramusculaires (50) et des fils (40) pour électrodes percutanés associés, ledit moyen (120) de mesure d'impédance fournissant un signal de contre-réaction (122) à une unité centrale (62) dudit générateur (10) de train d'impulsions indiquant les changements d'impédance de ladite électrode (50) et du fil (40) pour électrode associé.

10. Système de stimulation intramusculaire percutané selon la revendication 1, dans lequel ladite mémoire non volatile (66, 68) comprend en outre des données de retard de session de stimulation indiquant un intervalle de temps choisi après lequel une session de train d'impulsions de stimulation doit commencer en fonction des paramètres de train d'impulsions de stimulation enregistrés.
